# EUROPEAN PATENT APPLICATION

(11) **EP 3 647 791 A2**
(43) Date of publication of application: **06.05.2020**
(21) Application number: 19206031.7
(22) Date of filing: 29.10.2019
(51) Int. Cl.: G01N 33/68

(54) **SRM/MRM ASSAYS FOR NOTCH PATHWAY PROTEINS**

(30) Priority: 30.10.2018 US 201862752842 P
(71) Applicant: Expression Pathology, Inc., Rockville, MD 20850 (US)
(72) Inventor: HEMBROUGH, Todd, Gaithersburg, MD 20882 (US); CECCHI, Fabiola, Potomac, MD 20854 (US); SCHWARTZ, Sarit, Rockville, MD 20852 (US); SCOTT, Kerry, Germantown, MD 20874 (US)
(74) Representative: AWA Sweden AB

(57) **Abstract**

Methods are provided for detecting and quantifying the DLL1, DLL3, JAG1, JAG2, Notchl, Notch 2, Notch 3, Notch 4, and/or DLL4 proteins in biological samples, such as a formalin fixed paraffin embedded tissue samples, using mass spectrometry. Additionally, methods are provided for treating cancer based upon the level of the DLL1, DLL3, JAG1, JAG2, Notchl, Notch 2, Notch 3, Notch 4, and/or DLL4 proteins in the biological samples.

SEQUENCE LISTING

<110>  Expression Pathology Inc

<120>  SRM/MRM ASSAYS FOR NOTCH PATHWAY

<130>  EP-21110441

<150>  62/752842
<151>  2018-10-30

<160>  18

<170>  PatentIn version 3.5

<210>  1
<211>  13
<212>  PRT
<213>  Homo sapiens

<400>  1

Tyr Pro Ala Val Asp Tyr Asn Leu Val Gln Asp Leu Lys
1               5                   10


<210>  2
<211>  17
<212>  PRT
<213>  Homo sapiens

<400>  2

His Leu Thr Val Gly Glu Glu Trp Ser Gln Asp Leu His Ser Ser Gly
1               5                   10                  15


Arg




<210>  3
<211>  7
<212>  PRT
<213>  Homo sapiens

<400>  3

Ala Gly Ala Trp Glu Leu Arg
1               5


<210>  4
<211>  9
<212>  PRT
<213>  Homo sapiens

<400>  4

Leu Ala Ala Gly Gly Pro Trp Ala Arg
1               5

<210>  5
<211>  10
<212>  PRT
<213>  Homo sapiens

<400>  5

Asp Leu Glu Ser Ala Gln Ser Leu Asn Arg
1               5                   10


<210>  6
<211>  9
<212>  PRT
<213>  Homo sapiens

<400>  6

Gln Pro Ala Tyr Thr Leu Val Asp Arg
1               5


<210>  7
<211>  15
<212>  PRT
<213>  Homo sapiens

<400>  7

Ala Asp Glu Ala Leu Pro Gly Pro Ala Gly His Ala Ala Val Arg
1               5                   10                  15


<210>  8
<211>  10
<212>  PRT
<213>  Homo sapiens

<400>  8

Glu Asp Glu Glu Asp Glu Asp Leu Gly Arg
1               5                   10


<210>  9
<211>  9
<212>  PRT
<213>  Homo sapiens

<400>  9

Leu Ala Phe Glu Thr Gly Pro Pro Arg
1               5


<210>  10
<211>  9
<212>  PRT
<213>  Homo sapiens

<400>  10

Leu Leu Asp Glu Tyr Asn Leu Val Arg

1                   5


<210>  11
<211>  11
<212>  PRT
<213>  Homo sapiens

<400>  11

Glu Pro Val Gly Gln Asp Ala Val Gly Leu Lys
1               5                   10


<210>  12
<211>  11
<212>  PRT
<213>  Homo sapiens

<400>  12

Ala Leu Gly Thr Leu Leu His Thr Asn Leu Arg
1               5                   10


<210>  13
<211>  9
<212>  PRT
<213>  Homo sapiens

<400>  13

Ala Gly Leu Gly Pro Gln Gly Pro Arg
1               5


<210>  14
<211>  9
<212>  PRT
<213>  Homo sapiens

<400>  14

Glu Ala Ala Ala Gln Ile Gly Val Arg
1               5


<210>  15
<211>  10
<212>  PRT
<213>  Homo sapiens

<400>  15

Glu Gln Thr Pro Leu Phe Leu Ala Ala Arg
1               5                   10


<210>  16
<211>  14
<212>  PRT
<213>  Homo sapiens

<400>  16

Ser Leu Ser Gly Val Gly Ala Gly Gly Gly Pro Thr Pro Arg
1               5                   10

<210>  17
<211>  10
<212>  PRT
<213>  Homo sapiens

<400>  17

Asp Asn Leu Ile Pro Ala Ala Gln Leu Lys
1               5                   10

<210>  18
<211>  17
<212>  PRT
<213>  Homo sapiens

<400>  18

Gln Gln Asn His Thr Leu Asp Tyr Asn Leu Ala Pro Gly Pro Leu Gly
1               5                   10                  15

Arg

## Description

### Background

The Notch signaling pathway drives proliferation, differentiation, apoptosis, cell fate, and maintenance of stem cells in several normal tissues and various cancers. The Notch signal pathway is most active during embryonic development. Aberrant activation of Notch signaling has been described in several tumor types and activated Notch pathway proteins have been associated with cancer progression and therapeutic resistance.

DLL1 (also known as delta-like protein 1) is a member of the delta/serrate/jagged family of proteins. It plays a role in mediating cell fate decisions during hematopoiesis and may play a role in cell-to-cell communication. DLL1 is overexpressed in many cancers and inhibitors of DLL1 function are in development for cancer therapy. Accordingly, an assay that can quantitate the DLL1 protein in tumor cells is useful as part of a cancer therapy regimen and, in particular, information about the level of DLL1 expression can be used in selecting specific methods of treatment.

DLL3 (also known as Delta-like 3) that is normally expressed in fetal brain at a specific time in neurological development. During this time it inhibits both cis- and transacting Notch pathway activation by interacting with Notch and DLL1 and redirecting or retaining them to late endosomal/lysosomal compartments or the Golgi, respectively, thereby preventing their localization to the cell surface. Moreover, DLL3 is one of several Notch ligands that appear to be direct downstream targets of the ASCL1 protein. Together, these observations suggest that DLL3 might be associated with the neuroendocrine phenotype and contributes to neuroendocrine tumorigenesis. A number of DLL3 inhibitors have been developed and are being tested for treatment of neuroendocrine tumors. The expression pattern of the DLL3 protein is relevant to the identification of tumors and choice of cancer therapy, including diagnosis and treatment of high-grade pulmonary neuroendocrine tumors, small cell lung cancer (SCLC) and large cell neuroendocrine carcinoma (LCNEC). Accordingly, an assay that can quantitate the DLL1 protein in tumor cells is useful as part of a cancer therapy regimen and, in particular, information about the level of DLL1 expression can be used in selecting specific methods of treatment.

JAG1 (also known as jagged1) is a cell surface protein (ligand) that interacts with receptors in the mammalian Notch signaling pathway. The Notch Signaling Pathway is a highly conserved pathway that functions to establish and regulate cell fate decisions in many organ systems. Once the JAG1-NOTCH (receptor-ligand) interactions take place, a cascade of proteolytic cleavages is triggered resulting in activation of transcription for downstream target genes that can initiate and promote the cancer process. Cancer therapeutic agents have been developed that prevent the interaction between JAG1 and its Notch receptors and, accordingly, an assay that can quantitate the JAG1 protein in tumor cells can be used in selecting methods of cancer treatment.

JAG2 (also known as jagged2) is a cell surface protein (ligand) that interacts with receptors in the mammalian Notch signaling pathway. The most well-understood function of JAG2 is to interact with the Notch2 receptor to activate the Notch signaling pathway, which can result in the initiation and progression of the cancer process. Cancer therapeutic agents have been developed that prevent the interaction between JAG2 and the Notch2 receptor and , accordingly, an assay that can quantitate the JAG2 protein in tumor cells can be used in selecting methods of cancer treatment.

Notch1, Notch2, Notch3, and Notch4 are a family of transmembrane proteins with repeated extracellular EGF domains and the notch (or DSL) domains. These proteins are involved in lateral inhibition in embryogenesis and, when aberrantly expressed, drive the growth of cancer cells. The Notch signaling network is an evolutionarily conserved intercellular signaling pathway that regulates interactions between physically adjacent cells. Members of this Type 1 transmembrane protein family share structural characteristics including an extracellular domain consisting of multiple epidermal growth factor-like (EGF) repeats, and an intracellular domain consisting of multiple, different domain types. These proteins are cleaved in the trans-Golgi network, and presented on the cell surface as a heterodimer where they function as receptors for membrane bound ligands that play a role in vascular, renal and hepatic development. Multiple Notch receptor inhibitors have been developed, or are being developed, as targeted cancer therapeutic agents, accordingly, an assay that can quantitate each of the 4 Notch receptors can be used in selecting methods of cancer treatment.

DLL4 (also known as delta like canonical Notch ligand 4) is a protein that functions as a Notch ligand characterized by a DSL domain, EGF repeats, and a transmembrane domain. DLL4 activates NOTCH1 and NOTCH4. DLL4 is involved in angiogenesis and negatively regulates endothelial cell proliferation, migration, and angiogenic sprouting. DLL4 is essential for retinal progenitor proliferation and is required for suppressing rod fates in late retinal progenitors as well as for proper generation of other retinal cell types. DLL4 expression has been implicated in driving the tumor process and as a result multiple DLL4 inhibitors are being developed. In light of this, an assay that can quantitate the JAG2 protein in tumor cells can be used in selecting methods of cancer treatment.

### Summary

The level of protein expression of one or more of the proteins involved in the Notch signaling pathway comprising DLL1, DLL3, JAG1, JAG2, Notch1, Notch 2, Notch 3, Notch 4, and/or DLL4 in patient tumor tissue can be determined by quantitating a peptide derived from subsequences of each of the full-length proteins. Each peptide can be detected using mass spectrometry techniques, such as spectrometry-based Selected Reaction Monitoring (SRM), also referred to as Multiple Reaction Monitoring (MRM), referred to herein as an SRM/MRM assay. An SRM/MRM assay is used to quantitate the amount of a fragment peptide, directly in cells procured from cancer patient tissue, such as, for example formalin fixed cancer tissue.

The quantitation can be relative or absolute. When absolute quantitation is required the measured level of each peptide is compared to a known amount of a labeled reference peptide having the same amino acid sequence as the measured peptide. The peptides are unique to a specific protein and, accordingly, one peptide molecule is derived from one protein molecule and a measure of the molar amount of peptide provides a direct measurement of the molar amount of the protein from which it was derived. The measurements of protein expression can be used for diagnosis of cancer, staging of the cancer, prognosis of cancer progression, predicting the likelihood of clinical response to various cancer treatments and therapies, and the like.

### Detailed Description

Methods are provided for quantitative proteomics-based assays that quantify the DLL1, DLL3, JAG1, JAG2, Notch1, Notch 2, Notch 3, Notch 4, and/or DLL4 proteins in formalin-fixed tissues from cancer patients. Data from these assays can be used to make improved treatment decisions for cancer therapy, for example.

The SRM/MRM assays can be used to measure relative or absolute quantitative levels of the peptides from the DLL1, DLL3, JAG1, JAG2, Notch1, Notch 2, Notch 3, Notch 4, and/or DLL4 proteins in a given protein preparation obtained from a biological sample. More specifically, the SRM/MRM assay can measure these peptides directly in complex protein lysate samples prepared from cells procured from patient tissue samples, such as formalin fixed cancer patient tissue. Methods of preparing protein samples from formalin-fixed tissue are described in U.S. Patent No. 7,473,532, the contents of which are hereby incorporated by references in their entirety. The methods described in U.S. Patent No. 7,473,532 may conveniently be carried out using Liquid Tissue® reagents and protocol available from Expression Pathology Inc. (Rockville, MD).

The most widely and advantageously available form of tissues from cancer patient tissue is formalin-fixed, paraffin embedded tissue. Formaldehyde/formalin fixation of surgically removed tissue is far the most common method of preserving cancer tissue samples worldwide and is the accepted convention for standard pathology practice. Aqueous solutions of formaldehyde are referred to as formalin. "100%" formalin consists of a saturated solution of formaldehyde (about 40% by volume or 37% by mass) in water, with a small amount of stabilizer, usually methanol to limit oxidation and degree of polymerization. The most common way in which tissue is preserved is to soak whole tissue for extended periods of time (8 hours to 48 hours) in aqueous formaldehyde, commonly termed 10% neutral buffered formalin, followed by embedding the fixed whole tissue in paraffin wax for long term storage at room temperature. Thus molecular analytical methods to analyze formalin fixed cancer tissue will be the most accepted and heavily utilized methods for analysis of cancer patient tissue.

Results from the SRM/MRM assay can be used to correlate accurate and precise quantitative levels of the DLL1, DLL3, JAG1, JAG2, Notch1, Notch 2, Notch 3, Notch 4, and/or DLL4 proteins within the specific tissue samples (e.g., cancer tissue sample) of the patient or subject from whom the tissue (biological sample) was collected and preserved. This not only provides diagnostic information about the cancer, but can be used as part of improved methods of treatment for the patient. Such an assay that provides diagnostically and therapeutically important information about levels of protein expression in a diseased tissue or other patient sample is termed a companion diagnostic assay. For example, the assay can be designed to diagnose the stage or degree of a cancer and determine a therapeutic agent to which a patient is most likely to respond.

The assay described herein measures relative or absolute levels of peptides from the DLL1, DLL3, JAG1, JAG2, Notch1, Notch 2, Notch 3, Notch 4, and/or DLL4 proteins. Relative quantitative level of a given protein is determined by the SRM/MRM methodology, for example, by comparing SRM/MRM signature peak areas (e.g., signature peak area or integrated fragment ion intensity) of an individual fragment peptide derived from a protein in different samples. Alternatively, it is possible to compare multiple SRM/MRM signature peak areas for multiple signature peptides, where each peptide has its own specific SRM/MRM signature peak, to determine the relative protein content in one biological sample with the content of the same protein(s) in one or more additional or different biological samples. In this way, the amount of a particular peptide, or peptides, from the subject protein(s), and therefore the amount of the designated protein(s), is determined relative to the same peptide, or peptides, across two or more biological samples under the same experimental conditions. In addition, relative quantitation can be determined for a given peptide, or peptides, from a given protein within a single sample by comparing the signature peak area for that peptide by SRM/MRM methodology to the signature peak area for another and different peptide, or peptides, from a different protein, or proteins, within the same protein preparation from the biological sample. In this way, the amount of a particular peptide from a designated protein, and therefore the amount of that protein, is determined relative one to another within the same sample. These approaches generate quantitation of an individual peptide, or peptides, from a designated protein to the amount of another peptide, or peptides, between samples and within samples wherein the amounts as determined by signature peak area are relative one to another, regardless of the absolute weight to volume or weight to weight amounts of the selected peptide in the protein preparation from the biological sample. Relative quantitative data about individual signature peak areas between different samples are normalized to the amount of protein analyzed per sample. Relative quantitation can be performed across many peptides from multiple proteins and one or more of the designated proteins simultaneously in a single sample and/or across many samples to gain insight into relative protein amounts, such as one peptide/protein with respect to other peptides/proteins.

Absolute quantitative levels of the designated protein are determined comparing the SRM/MRM signature peak area of an individual peptide from the designated protein in a biological sample to the SRM/MRM signature peak area of a spiked internal standard. In one embodiment, the internal standard is a synthetic version of the same exact peptide derived from the designated protein that contains one or more amino acid residues labeled with one or more heavy isotopes. Such isotope labeled internal standards are synthesized so that when analyzed by mass spectrometry a standard generates a predictable and consistent SRM/MRM signature peak that is different and distinct from the native peptide signature peak and which can be used as a comparator peak. Thus when the internal standard is spiked into a protein preparation from a biological sample in known amounts and analyzed by mass spectrometry, the SRM/MRM signature peak area of the native peptide is compared to the SRM/MRM signature peak area of the internal standard peptide, and this numerical comparison indicates either the absolute molarity and/or absolute weight of the native peptide present in the original protein preparation from the biological sample. Absolute quantitative data for fragment peptides are displayed according to the amount of protein analyzed per sample. Absolute quantitation can be performed across many peptides, and thus proteins, simultaneously in a single sample and/or across many samples to gain insight into absolute protein amounts in individual bio logical samples and in entire cohorts of individual samples.

The SRM/MRM assay method can be used to aid diagnosis of the stage of cancer, for example, directly in patient-derived tissue, such as formalin fixed tissue, and as part of improved methods of treatment by helping to determine which therapeutic agent would be most advantageous for use in treating that patient. Cancer tissue that is removed from a patient either through surgery, such as for therapeutic removal of partial or entire tumors, or through biopsy procedures conducted to determine the presence or absence of suspected disease, is analyzed to determine whether or not a specific protein, or proteins, and which forms of proteins, are present in that patient tissue. Moreover, the expression level of a protein, or multiple proteins, can be determined and compared to a "normal" or reference level found in healthy tissue. Normal or reference levels of proteins found in healthy tissue may be derived from, for example, the relevant tissues of one or more individuals that do not have cancer. Alternatively, normal or reference levels may be obtained for individuals with cancer by analysis of relevant tissues not affected by the cancer. Assays of protein levels from one, some, or all of the designated proteins can also be used to diagnose the stage of cancer in a patient or subject diagnosed with cancer by employing the protein levels. The level of an individual peptide derived from a designated protein is defined as the molar amount of the peptide determined by the SRM/MRM assay per total amount of protein lysate analyzed. Information regarding a designated protein or proteins can thus be used to aid in determining the stage or grade of a cancer by correlating the level of the protein(s) (or fragment peptides from the proteins) with levels observed in normal tissues. Once the quantitative amount of one or more of the designated proteins has been determined in the cancer cells, that information can be matched to a list of therapeutic agents (chemical and biological) developed to specifically treat cancer tissue that is characterized by, for example, abnormal expression of the protein or protein(s) that were assayed. Matching information from a protein assay to a list of therapeutic agents that specifically targets, for example, the designated protein or cells/tissue expressing the protein, defines what may be termed a personalized medicine approach to treating disease. The assay methods described herein form the foundation of a personalized medicine approach by using analysis of proteins from the patient's own tissue as a source for diagnostic and treatment decisions.

Suitable fragment peptides derived from a designated protein may be generated by a variety of means including by the use of the Liquid Tissue® protocol provided in US Patent 7,473,532. The Liquid Tissue® protocol and reagents are capable ofproducing peptide samples suitable for mass spectroscopic analysis from formalin fixed paraffin embedded tissue by proteolytic digestion of the proteins in the tissue/biological sample. In the Liquid Tissue® protocol the tissue/biological sample is heated in a buffer for an extended period of time (e.g., from about 80° C to about 100° C for a period of time from about 10 minutes to about 4 hours) to reverse or release protein cross-linking. The buffer employed is a neutral buffer, (e.g., a Tris-based buffer, or a buffer containing a detergent). Following heat treatment the tissue/biological sample is treated with one or more proteases, including but not limited to trypsin, chymotrypsin, pepsin, and endoproteinase Lys-C for a time sufficient to disrupt the tissue and cellular structure of said biological sample. The result of the heating and proteolysis is a liquid, soluble, dilutable biomolecule lysate.

In principle, any predicted peptide derived from a designated protein, prepared for example by digesting with a protease of known specificity (e.g. trypsin), can be used as a surrogate reporter to determine the abundance of a designated protein in a sample using a mass spectrometry-based SRM/MRM assay. Similarly, any predicted peptide sequence containing an amino acid residue at a site that is known to be potentially modified in the designated protein also might potentially be used to assay the extent of modification of the designated protein in a sample.

Surprisingly, however, it was found that many potential peptide sequences from the proteins listed above are unsuitable or ineffective for use in mass spectrometry-based SRM/MRM assays for reasons that are not immediately evident. As it was not possible to predict suitable peptides for MRM/SRM assay, it was necessary to experimentally identify modified and unmodified peptides in actual Liquid Tissue® lysates to develop a reliable and accurate SRM/MRM assay for each designated protein. While not wishing to be bound by any theory, it is believed that some peptides might, for example, be difficult to detect by mass spectrometry because they do not ionize well or produce fragments distinct from other proteins. Peptides may also fail to resolve well in separation (e.g., liquid chromatography), or may adhere to glass or plastic ware.

The peptides found in Table 1 were derived from the DLL1, DLL3, JAG1, JAG2, Notch1, Notch 2, Notch 3, Notch 4, and/or DLL4 proteins by protease digestion of all the proteins within a complex Liquid Tissue® lysate prepared from cells procured from formalin fixed cancer tissue. In some embodiments, the cancer tissue is a tumor, such as a primary or secondary tumor. Unless noted otherwise, in each instance the protease was trypsin. The Liquid Tissue® lysate was then analyzed by mass spectrometry to determine those peptides derived from a designated protein that are detected and analyzed by mass spectrometry. Identification of a specific preferred subset of peptides for mass spectrometric analysis is based on: 1) experimental determination of which peptide or peptides from a protein ionized in mass spectrometry analyses of Liquid Tissue® lysates; and 2) the ability of the peptide to survive the protocol and experimental conditions used in preparing a Liquid Tissue® lysate. This latter property extends not only to the amino acid sequence of the peptide, but also to the ability of a modified amino acid residue within a peptide to survive in modified form during the sample preparation.

### Examples

Protein lysates from cells procured directly from formalin (formaldehyde) fixed tissue were prepared using the Liquid Tissue® reagents and protocol that entails collecting cells into a sample tube via tissue microdissection followed by heating the cells in the Liquid Tissue® buffer for an extended period of time. Once the formalin-induced cross linking has been negatively affected, the tissue/cells were then digested to completion in a predictable manner using a protease, as for example including but not limited to the protease trypsin. Each protein lysate was reduced to a collection of peptides by digestion of intact polypeptides with the protease. Each Liquid Tissue® lysate was analyzed (e.g., by ion trap mass spectrometry) to perform multiple global proteomic surveys of the peptides where the data was presented as identification of as many peptides as could be identified by mass spectrometry from all cellular proteins present in each protein lysate. An ion trap mass spectrometer or another form of a mass spectrometer that is capable of performing global profiling for identification of as many peptides as possible from a single complex protein/peptide lysate is typically employed.

Although an SRM/MRM assay can be developed and performed on any type of mass spectrometer, including a MALDI, ion trap, or triple quadrupole, advantageously a triple quadrupole instrument platform is used. This type of a mass spectrometer is able to analyze a single isolated target peptide within a very complex protein lysate that may consist of hundreds of thousands to millions of individual peptides from all the proteins contained within a cell. The described method was used to: 1) identify candidate peptides from each designated protein that can be used for a mass spectrometry-based SRM/MRM assay for the designated protein, 2) develop an individual SRM/MRM assay, or assays, for target peptides from the designated protein in order to correlate and 3) apply quantitative assays to cancer diagnosis and/or choice of optimal therapy.

For the Notch protein assays, once as many peptides as possible were identified in a single MS analysis of a single lysate under the conditions employed, then that list of peptides was collated and used to determine the proteins that were detected in that lysate. That process was repeated for multiple Liquid Tissue® lysates, and the very large list of peptides was collated into a single dataset. That type of dataset can be considered to represent the peptides that can be detected in the type of biological sample that was analyzed (after protease digestion), and specifically in a Liquid Tissue® lysate of the biological sample, and thus includes the peptides for each of the designated proteins.

The tryptic peptides identified as useful in the determination of absolute or relative amounts of the DLL1, DLL3, JAG1, JAG2, Notch1, Notch 2, Notch 3, Notch 4, and/or DLL4 proteins are listed in Table 1. Each of these peptides was detected by mass spectrometry in Liquid Tissue® lysates prepared from formalin fixed, paraffin embedded tissue. Thus, each peptide can be used in a quantitative SRM/MRM assay for a designated protein in human biological samples, including directly in formalin fixed patient tissue.

**Table 1**

| **Protein** | **SEQ ID NO** | **Peptide Sequence** |
|---|---|---|
| DLL1 | 1 | YPAVDYNLVQDLK |
| DLL1 | 2 | HLTVGEEWSQDLHSSGR |
| DLL3 | 3 | AGAWELR |
| DLL3 | 4 | LAAGGPWAR |
| JAG1 | 5 | DLESAQSLNR |
| JAG1 | 6 | QPAYTLVDR |
| JAG2 | 7 | ADEALPGPAGHAAVR |
| JAG2 | 8 | EDEEDEDLGR |
| Notch1 | 9 | LAFETGPPR |
| Notch1 | 10 | LLDEYNLVR |
| Notch2 | 11 | EPVGQDAVGLK |
| Notch2 | 12 | ALGTLLHTNLR |
| Notch3 | 13 | AGLGPQGPR |
| Notch3 | 14 | EAAAQIGVR |
| Notch4 | 15 | EQTPLFLAAR |
| Notch4 | 16 | SLSGVGAGGGPTPR |
| DLL4 | 17 | DNLIPAAQLK |
| DLL4 | 18 | QQNHTLDYNLAPGPLGR |

The tryptic peptides listed in Table 1 typically were detected from multiple Liquid Tissue® lysates of multiple different formalin fixed tissues of different human organs including, for example, prostate, colon, and breast.

### Assay Method

1. Identification of SRM/MRM candidate fragment peptides for a protein
   a. Prepare a Liquid Tissue® protein lysate from a formalin fixed biological sample using a protease or proteases, (that may or may not include trypsin), to digest proteins
   b. Analyze all protein fragments in the Liquid Tissue® lysate on an ion trap tandem mass spectrometer and identify all fragment peptides from a designated protein, where individual fragment peptides do not contain any peptide modifications such as phosphorylations or glycosylations
   c. Analyze all protein fragments in the Liquid Tissue® lysate on an ion trap tandem mass spectrometer and identify all fragment peptides from the protein that carry peptide modifications such as for example phosphorylated or glycosylated residues
   d. All peptides generated by a specific digestion method from an entire, full length protein can potentially be measured, but preferred peptides used for development of the SRM/MRM assay are those that are identified by mass spectrometry directly in a complex Liquid Tissue® protein lysate prepared from a formalin fixed biological sample
2. Mass Spectrometry Assay for Fragment Peptides from a Designated Protein
   a. SRM/MRM assay on a triple quadrupole mass spectrometer for individual fragment peptides identified in a Liquid Tissue® lysate is applied to peptides from the protein:
      i. Determine optimal retention time for a fragment peptide for optimal chromatography conditions including but not limited to gel electrophoresis, liquid chromatography, capillary electrophoresis, nano-reversed phase liquid chromatography, high performance liquid chromatography, or reverse phase high performance liquid chromatography.
      ii. Determine the mono isotopic mass of the peptide, the precursor charge state for each peptide, the precursor m/z value for each peptide, the m/z transition ions for each peptide, and the ion type of each transition ion for each fragment peptide in order to develop an SRM/MRM assay for each peptide.
      iii. SRM/MRM assay can then be conducted using the information from (i) and (ii) on a triple quadrupole mass spectrometer where each peptide has a characteristic and unique SRM/MRM signature peak that precisely defines the unique SRM/MRM assay as performed on a triple quadrupole mass spectrometer.
   b. Perform SRM/MRM analysis so that the amount of the fragment peptide of the protein that is detected, as a function of the unique SRM/MRM signature peak area from an SRM/MRM mass spectrometry analysis, can indicate both the relative and absolute amount of the protein in a particular protein lysate.
      i. Relative quantitation may be achieved by:
         1. Determining increased or decreased presence of the protein by comparing the SRM/MRM signature peak area from a given fragment peptide detected in a Liquid Tissue® lysate from one formalin fixed biological sample to the same SRM/MRM signature peak area of the same fragment peptide in at least a second, third, fourth or more Liquid Tissue® lysates from least a second, third, fourth or more formalin fixed biological samples.
         2. Determining increased or decreased presence of the protein by comparing the SRM/MRM signature peak area from a given fragment peptide detected in a Liquid Tissue® lysate from one formalin fixed biological sample to SRM/MRM signature peak areas developed from fragment peptides from other proteins, in other samples derived from different and separate biological sources, where the SRM/MRM signature peak area comparison between the 2 samples for a peptide fragment are normalized to amount of protein analyzed in each sample.
         3. Determining increased or decreased presence of the protein by comparing the SRM/MRM signature peak area for a given fragment peptide to the SRM/MRM signature peak areas from other fragment peptides derived from different proteins within the same Liquid Tissue® lysate from the formalin fixed biological sample in order to normalize changing levels of a protein to levels of other proteins that do not change their levels of expression under various cellular conditions.
         4. These assays can be applied to both unmodified fragment peptides and for modified fragment peptides of the protein, where the modifications include but are not limited to phosphorylation and/or glycosylation, and where the relative levels of modified peptides are determined in the same manner as determining relative amounts of unmodified peptides.
      ii. Absolute quantitation of a given peptide may be achieved by comparing the SRM/MRM signature peak area for a given fragment peptide from the designated protein in an individual biological sample to the SRM/MRM signature peak area of an internal fragment peptide standard spiked into the protein lysate from the biological sample:
         1. The internal standard is a labeled synthetic version of the fragment peptide from the designated protein that is being interrogated. This standard is spiked into a sample in known amounts, and the SRM/MRM signature peak area can be determined for both the internal fragment peptide standard and the native fragment peptide in the biological sample separately, followed by comparison of both peak areas.
         2. This can be applied to unmodified fragment peptides and modified fragment peptides, where the modifications include but are not limited to phosphorylation and/or glycosylation, and where the absolute levels of modified peptides can be determined in the same manner as determining absolute levels of unmodified peptides.
3. Apply Fragment Peptide Quantitation to Cancer Diagnosis and Treatment
   a. Perform relative and/or absolute quantitation of fragment peptide levels of the designated protein and demonstrate that the previously-determined association, as well understood in the field of cancer, of expression of the designated protein to the stage/grade/status of cancer in patient tumor tissue is confirmed.
   b. Perform relative and/or absolute quantitation of fragment peptide levels of the designated protein and demonstrate correlation with clinical outcomes from different treatment strategies, wherein this correlation has already been demonstrated in the field or can be demonstrated in the future through correlation studies across cohorts of patients and tissue from those patients. Once either previously established correlations or correlations derived in the future are confirmed by this assay then the assay method can be used to determine optimal treatment strategy

Specific and unique characteristics about specific fragment peptides from each designated protein were developed by analysis of all fragment peptides on both an ion trap and triple quadrupole mass spectrometers. That information must be determined experimentally for each and every candidate SRM/MRM peptide directly in Liquid Tissue® lysates from formalin fixed samples/tissue; because, interestingly, not all peptides from any designated protein can be detected in such lysates using SRM/MRM as described herein, indicating that fragment peptides not detected cannot be considered candidate peptides for developing an SRM/MRM assay for use in quantitating peptides/proteins directly in Liquid Tissue® lysates from formalin fixed samples/tissue.

A particular SRM/MRM assay for a fragment peptide is performed on a triple quadrupole mass spectrometer. An experimental sample analyzed by a particular protein SRM/MRM assay is for example a Liquid Tissue® protein lysate prepared from a tissue that had been formalin fixed and paraffin embedded. Data from such as assay indicates the presence of the unique SRM/MRM signature peak for this fragment peptide in the formalin fixed sample.

Specific transition ion characteristics for this peptide are used to quantitatively measure a particular fragment peptide in formalin fixed biological samples. These data indicate absolute amounts of this fragment peptide as a function of the molar amount of the peptide per microgram of protein lysate analyzed. Assessment of corresponding protein levels in tissues based on analysis of formalin fixed patient-derived tissue can provide diagnostic, prognostic, and therapeutically-relevant information about each particular patient. In one embodiment, this disclosure describes a method for measuring the level of each of the proteins listed in Table 1 in a biological sample, comprising detecting and/or quantifying the amount of one or more modified or unmodified fragment peptides in a protein digest prepared from said biological sample using mass spectrometry; and calculating the level of modified or unmodified protein in said sample; and wherein said level is a relative level or an absolute level. In a related embodiment, quantifying one or more fragment peptides comprises determining the amount of the each of the fragment peptides in a biological sample by comparison to an added internal standard peptide of known amount, wherein one or more of the fragment peptides in the biological sample is compared to an internal standard peptide having the same amino acid sequence. In some embodiments the internal standard is an isotopically labeled internal standard peptide comprising one or more heavy stable isotopes selected from ¹⁸O, ¹⁷O, ³⁴S, ¹⁵N, ¹³C, ²H or combinations thereof.

The method for measuring the level of a designated protein a biological sample described herein (or fragment peptides as surrogates thereof) may be used as a diagnostic indicator of cancer in a patient or subject. In one embodiment, the results from measurements of the level of a designated protein may be employed to determine the diagnostic stage/grade/status of a cancer by correlating (e.g., comparing) the level of the protein found in a tissue with the level of that protein found in normal and/or cancerous or precancerous tissues.

Because both nucleic acids and protein can be analyzed from the same Liquid Tissue® biomolecular preparation it is possible to generate additional information about disease diagnosis and drug treatment decisions from the nucleic acids in the same sample used for proteomic analysis. For example, if a designated protein is expressed by certain cells at increased levels, when assayed by SRM the data can provide information about the state of the cells and their potential for uncontrolled growth, potential drug resistance and the development of cancers can be obtained. At the same time, information about the status of the corresponding genes and/or the nucleic acids and proteins they encode *(e.g*., mRNA molecules and their expression levels or splice variations) can be obtained from nucleic acids present in the same Liquid Tissue® biomolecular preparation can be assessed simultaneously to the SRM analysis of the designated protein. Any gene and/or nucleic acid not from the designated protein and which is present in the same biomolecular preparation can be assessed simultaneously to the SRM analysis of the designated protein. In one embodiment, information about the designated protein and/or one, two, three, four or more additional proteins may be assessed by examining the nucleic acids encoding those proteins. Those nucleic acids can be examined, for example, by one or more, two or more, or three or more of: sequencing methods, polymerase chain reaction methods, restriction fragment polymorphism analysis, identification of deletions, insertions, and/or determinations of the presence of mutations, including but not limited to, single base pair polymorphisms, transitions, transversions, or combinations thereof.

## Claims

1. A method for measuring the level of protein in a biological sample of formalin fixed tissue, the method comprising
detecting and/or quantifying by mass spectrometry an amount of one or more modified or unmodified fragment peptides derived from the protein in a protein digest prepared from said biological sample; and calculating the level of said protein in said sample;
wherein said protein is selected from the group consisting of DLL1, DLL3, JAG1, JAG2, Notch1, Notch 2, Notch 3, Notch 4, and DLL4 protein.

2. The method of claim 1, further comprising the step of fractionating said protein digest prior to detecting and/or quantifying the amount of said one or more modified or unmodified fragment peptides.

3. The method of claim 2, wherein said fractionating step is selected from the group consisting of liquid chromatography, nanoreversed phase liquid chromatography, high performance liquid chromatography and reverse phase high performance liquid chromatography.

4. The method of claim 1, wherein said protein digest comprises a protease digest, such as a trypsin digest.

5. The method of claim 1, wherein said mass spectrometry comprises tandem mass spectrometry, ion trap mass spectrometry, triple quadrupole mass spectrometry, ion trap/quadrupole hybrid mass spectrometry, MALDI-TOF mass spectrometry, MALDI mass spectrometry, and/or time of flight mass spectrometry.

6. The method of claim 5, wherein a mode of mass spectrometry used is Selected Reaction Monitoring (SRM), Multiple Reaction Monitoring (MRM), multiple Selected Reaction Monitoring (mSRM), and/or intelligent Selected Reaction Monitoring (iSRM).

7. The method of claim 1, wherein said protein is DLL1 and said one or more fragment peptides are the peptides of SEQ ID NO:1 and/or SEQ ID NO:2, or
said protein is DLL3 and said one or more fragment peptides are the peptides of SEQ ID NO:3 and/or SEQ ID NO:4; or
said protein is JAG1 and said one or more fragment peptides are the peptides of SEQ ID NO:5 and/or SEQ ID NO:6; or
said protein is JAG2 and said one or more fragment peptides are the peptides of SEQ ID NO:7 and/or SEQ ID NO:8; or
said protein is Notch 1 and said one or more fragment peptides are the peptides of SEQ ID NO:9 and/or SEQ ID NO:10; or
said protein is Notch2 and said one or more fragment peptides are the peptides of SEQ ID NO:11 and/or SEQ ID NO:12; or
said protein is Notch3 and said one or more fragment peptides are the peptides of SEQ ID NO:13 and/or SEQ ID NO: 14; or
said protein is Notch4 and said one or more fragment peptides are the peptides of SEQ ID NO: 15 and/or SEQ ID NO: 16; or
said protein is DLL4 and said one or more fragment peptides are the peptides of SEQ ID NO:17 and/or SEQ ID NO:18.

8. The method of claim 1, wherein the tissue is paraffin embedded tissue.

9. The method of claim 1, wherein the tissue is obtained from a tumor, such as a primary or secondary tumor.

10. The method of claim 1, wherein quantifying said one or more fragment peptides comprises comparing the amount of said one or more fragment peptides in one biological sample to an amount of the same one or more fragment peptides in a different and separate biological sample.

11. The method of claim 1, wherein quantifying said one or more fragment peptides comprises determining the amount of said one or more fragment peptides in a biological sample by comparison to an added internal standard peptide of known amount having the same amino acid sequence.

12. The method of claim 11, wherein the internal standard peptide is an isotopically labeled peptide selected from ¹⁸O, ¹⁷O, ³⁴S, ¹⁵N, ¹³C, ²H and a combination thereof.

13. The method of claim 1, wherein detecting and/or quantifying the amount of the one or more fragment peptides in the protein digest indicates the presence of the corresponding protein and an association with a diagnostic stage/grade/status of cancer in a subject.

14. The method of claim 13, further comprising correlating results of said detecting and/or quantifying the amount of said one or more fragment peptides, or the level of the corresponding protein, to a cancer treatment therapy for the subject.

15. The method of claim 14, further comprising detecting and/or quantifying the amount of other proteins or peptides from other proteins in a multiplex format to provide additional information about a cancer treatment therapy for the subject.
